(19) Europäisches Patentamt
European Patent Office
Office européen des brevets

(11) Numéro de publication: **0 423 008 B1**

# (12) FASCICULE DE BREVET EUROPEEN

(45) Date de publication de fascicule du brevet: **28.12.94**

(51) Int. Cl.[5]: **C07C 68/06**, C07C 69/96

(21) Numéro de dépôt: **90402784.4**

(22) Date de dépôt: **08.10.90**

Le dossier contient des informations techniques présentées postérieurement au dépôt de la demande et ne figurant pas dans le présent fascicule.

## (54) Procédé de préparation de dicarbonate de dialcoyle secondaire ou tertiaire.

(30) Priorité: **09.10.89 FR 8913140**
**01.10.90 FR 9012056**

(43) Date de publication de la demande:
**17.04.91 Bulletin 91/16**

(45) Mention de la délivrance du brevet:
**28.12.94 Bulletin 94/52**

(84) Etats contractants désignés:
**AT BE CH DE DK ES FR GB GR IT LI LU NL SE**

(56) Documents cités:
**EP-A- 0 256 559**
**US-A- 3 078 294**

**CHEMICAL ABSTRACTS, vol. 81, no. 13, 30 septembre 1974 Columbus, Ohio, USA B.M.Pope: "Di-tert-butyl dicarbonate"**

**Organic Syntheses vol. 57, 1977, pages 45 - 50; B.M. Pope: "Di-tert-butyl**

**dicarbonate"**

(73) Titulaire: **RHONE-POULENC CHIMIE**
**25, Ouai Paul Doumer**
**F-92408 Courbevoie Cédex (FR)**

(72) Inventeur: **Desmurs, Jean**
**Route de Ternay,**
**La Jonquière**
**F-69360 Communay (FR)**
Inventeur: **Ratton, Serge**
**13, rue d'Ayen**
**F-78100 St-Germain-en-Laye (FR)**

(74) Mandataire: **Ricalens, François et al**
**RHONE-POULENC CHIMIE,**
**Direction de la Propriéte Industrielle,**
**25, quai Paul Doumer**
**F-92408 Courbevoie Cédex (FR)**

EP 0 423 008 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un nouveau procédé de préparation du dicarbonate de ditertiobutyle. Elle concerne plus particulièrement la préparation du dicarbonate de ditertiobutyle à partir de phosgène, de diphosgène ou de triphosgène.

Il est connu depuis longtemps de préparer le dicarbonate de ditertiobutyle, souvent dénommé (BOC)2O à partir du carbonate mixte de métal alcalin et de tertiobutyle et de phosgène comme cela est par exemple décrit par J.H. HOWE dans le journal of organic chemistry 27, 1901 (1962) ou dans son brevet US N° 3 078 294.

Il est aussi connu, selon l'article publié par Pozdev, Smirnova, Podgornova, Zentsova et le Kalei dans Zhurnal Urganicheskoï Khimii Vol. 15, N° 1, pages 106-109 en 1979 et traduit dans le Journal of Organic Chemistry USSR 1979, page 95, de préparer le dicarbonate de ditertiobutyle par condensation du carbonate de tertiobutyle avec un chlorure d'acide dans un mélange de toluène et de diméthylformamide. Lorsque nous reproduisons ces essais, les rendements obtenus en dicarbonate de ditertiobutyle ne sont pas suffisants.

La présente invention a permis de préparer le dicarbonate de ditertiobutyle directement à partir de dichlorure de diacide ou équivalent avec de bons rendements.

Elle consiste à condenser le carbonate mixte de métal alcalin et de tertiobutyle avec le dihalogénure de diacide ou équivalent en présence d'au moins un agent complexant.

La réaction peut être schématisée de la façon suivante :

$$2\ R_2C(R_1)(R_3)\text{-O-CO-OM} + (CO)_nX_2 \text{-->} [R_2C(R_1)(R_3)\text{-O-CO}]_2O + CO_2 + (n-1)CO + 2MX$$

avec X représentant un groupe partant choisi parmi les halogénures, de préférence chlorure et bromure ;avec $R_1$, $R_2$, $R_3$ étant aryle ou de préférence alcoyle (selon la définition du dictionnaire de chimie DUVAL) ou hydrogène, $R_1$, $R_2$, $R_3$ ne pouvant être simultanément hydrogène; $R_1$, $R_2$, $R_3$ étant avantageusement tels qu'un seul d'entre eux, de préférence aucun, ne soit hydrogène ; avec n représentant un entier choisi entre un et deux; avec $(CO)_nX_2$ représentant un dihalogènure d'oxalyle, un dihalogènure de carbonyle ou une de leur sources (telle que les polyphosgènes) et M représentant un métal alcalin de faible rayon atomique avantageusement le lithium et le sodium.

La somme des carbones de $R_1$, $R_2$ et $R_3$ est avantageusement au plus égale à 25, de préférence à 12.

Lorsque ledit dihalogénure de diacide est le phosgène, le diphosgène, le triphosgène, le agents complexants doivent posséder des propriétés complexantes élevées. Ainsi les amines décrites dans la demande de brevet européen déposée au nom de Mistubishi Chemical Industries limited et publiée sous le N° 0256 559 à savoir les mono amines tertiaires ou choisies parmi le 1,4-diazabicyclo (2,2,2) octane, le 1,8-diazabicyclo (5,4,0) undecene-7, l'hexamethyltetramine, la N-methyl piperidine la N-ethylpiperidine, la N-methylmorpholine, la morpholine, la N-N'-dialcoylpiperazine, la pyridine, la quinoléine et l'isoquinoléine, ont un pouvoir complexant insuffisant car ne répondant au contraintes de mobilité exposée ci-après.

Ainsi selon l'invention, il a été mis au point un procédé de péparation de dicarbonate (ou pyrocarbonate) de dialcoyle secondaire ou tertiaire où l'on met en contact un carbonate mixte d'alcoyle, secondaire ou tertiaire, et de metal alcalin avec un dihalogénure de diacide choisi parmi les halogénures de carbonyles et leurs di ou trimères et les halogénures d'oxalyle où ledit contact est mené en présence d'un agent complexant choisi parmi ceux possédant au moins une fonction amine ou possédant une fonction éther comportant au moins une autre fonction éther et parmi les cryptands,avec la condition que lorsque ledit dihalogénure de diacide est le phosgène, le diphosgène,et/ou le triphosgène, l'agent complexant n'est ni le dioxanne, ni une mono amine tertiaire, ni une amine choisie parmi le 1,4-diazabicyclo (2,2,2) octane, le 1,8-diazabicyclo (5,4,0) undecene-7 l'hexaméthyltétramine, la N-méthylpipéridine, la N-éthylpipéridine la N-méthylmorpholine, la morpholine, la N,N'-dialcoylpiperazine, la pyridine, la quinoléine et l'isoquinoléine.

Avantageusement X est choisi de manière que $R_2(R_1)(R_3)C\text{-O-COOH}$ présente un pKa supérieur à celui de HX, de préférence d'une valeur au moins égale à 2.

Le HX préféré présente un pKa au plus égal à 2 et est le plus souvent halohydrique, avantageusement bromhydrique ou surtout chlorhydrique, dans laquelle M représente un métal alcalin.

Le carbonate mixte de métal alcalin et de tertiobutyle est obtenu de manière en elle-même connue, par exemple par carbonatation de tertiobutylate.

Parmi les carbonates mixtes de métal alcalin et de tertiobutyle on prèfère utiliser les sels alcalins et tout particulièrement le sel de sodium (M = Na).

Les agents séquestrants utilisables sont avantageusement choisis d'une part parmi les amines et d'autre part parmi les éthers dont les molécules comportent au moins une autre fonction éther.

Ainsi, les agents séquestrants utilisables sont avantageusement choisis de manière qu'ils comportent, ou bien au moins une fonction amine; ou bien une fonction éther et au moins une fonction amine et/ou éther pour former un complexant avantageusement au moins bidenté, de préférence tridenté, les fonctions éther et/ou amine étant séparées par au moins 1, avantageusement 2 atomes et par au plus 4, avantageusement au plus 3 atomes, en général de carbone.

Lorsque les atomes réputés assurer la coordination sont reliés entre eux par 2 branches formant ainsi un cycle, il est préférable qu'une branche au moins comporte au moins 3 chaînons et l'autre au moins 2.

Les fonctions amine utilisables ne doivent pas être susceptibles de réagir avec le phosgène, les halogénures d'oxalyle ou équivalents, et sont de préférence tertiaires.

Les molécules complexantes, présentent avantageusement de 3 à 75 atomes de carbone de préférence de 6 à 39.

L'encombrement et la mobilité doivent être tels que les bi, tri ou polydentés soient complexants. Tel n'est pas le cas du 1,4 diaza (2,2,2) bicyclo octane ; ce qui explique le mauvais résultat obtenu (exemple B).

D'une manière générale, on peut quantifier cette contrainte en indiquant que les systèmes bicycliques présentant un nombre de chaînons au plus égal à 8, surtout lorsque les têtes de pont sont les atomes assurant la coordination, du type diaza bicyclo-octane-, heptane et inférieur et dans une moindre mesure nonane sont à éviter. D'une manière plus générale, il est avantageux d'éviter tout système bicyclique dont les têtes de pont sont des atomes prévus pour assurer la coordination et dont 2 des branches ont, compte non tenu des têtes de pont, une longueur de chaînons au plus égal à 2, de préférence au plus égal à 3 lorsque la troisième branche présente une longueur, exprimée en chaînon, inférieur à 7.

Le caractère bidenté au moins avec de préférence au moins une fonction amine est nécessaire pour le phosgène et dérivés, mais non pour l'halogénure d'oxalyle et équivalents.

On peut citer comme particulièrement intéressantes au moins 3 classes d'agents complexants comprenant les amines tertiaires oxygénées ; les polyéthers oxygénés ou soufrés, linéaires, cycliques ou macrocycliques ; les cryptands.

La première classe est constituée des agents séquestrants de formule générale :

$$N - [- CHR_1 -- CHR_2 -- O --(CHR_3 -- CHR_4 -- O)_n -- R_5]_3 \qquad (I)$$

dans laquelle n est un nombre entier supérieur ou égal à 0 et inférieur ou égal à 10 ($0 \leqq n \leqq 10$), $R_1$, $R_2$, $R_3$, $R_4$ identiques ou différents représentent un atome d'hydrogène ou un radical alkyle ayant de 1 à 4 atomes de carbone et $R_5$ représenteun radical alkyle ou cycloalkyle ayant de 1 à 12 atomes de carbone, un radical phényle ou un radical de formule $-C_mH_{2m}-\Phi$, ou $C_mH_{2m+1}-\Phi-$, m étant compris entre 1 et 12.

La deuxième classe d'agents complexants est constituée des polyéthers cycliques, de préférence macrocycliques, ayant de 6 à 30 atomes dans le cycle et de préférence de 15 à 30 atomes dans le cycle et constitués de 2 à 10, de préférence de 4 à 10, unités -O-X- dans lesquelles X est soit $-CHR_6-CHR_7-$ soit $-CHR_6-CHR_8-CR_9R_7-$, $R_6$, $R_7$, $R_8$ et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités -O-X- comprennent le groupement $-O-CHR_6-CHR_7$.

La troisième classe d'agents complexants est constituée des composés de formule générale :

$$R_{10}-Y[A - D]_p A - Y - R_{10} \qquad IIa$$

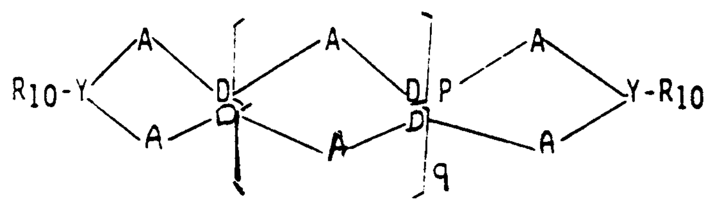

IIb

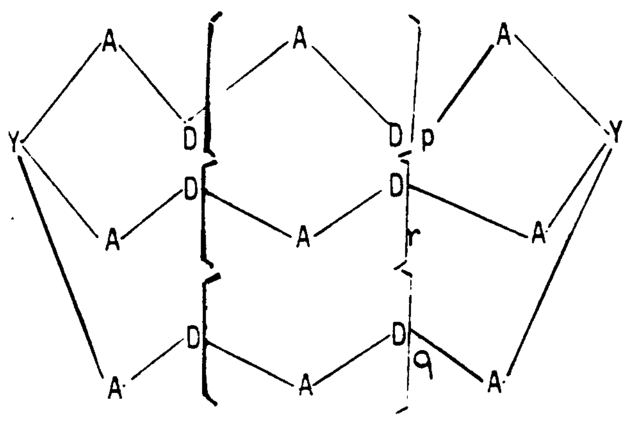

dans lesquelles

- Y représente O (divalent) , N ou P (trivalent) dans les formules IIa et IIb et N ou P dans la dernière formule.
- A représente un groupement alkylène ayant de 1 à 3 atomes de carbone.
- D représente 0, S ou N-R11 où R11 représente un radical alkyle ayant de 1 à 6 atomes de carbone.
- $R_{10}$ représente un radical alkyle ayant 1 à 6 atomes de carbone.
- lorsque dans les formule ci-dessus la valence des atomes Y n'est pas satifaite, des radicaux alkyles $R_{10}$ satisfont alors les dites valences.
- p, q et r identiques ou différents sont des nombres entiers compris entre 1 et 5.

Selon un mode de réalisation préférentiel du procédé de l'invention, on utilise au moins un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 6, et pour lesqules $R_5$ représente un radical alkyle ayant de 1 à 4 atomes de carbone.

On peut citer :

- la tris(oxa-3 butyl) amine de formule :

$N-(CH_2-CH_2-O-CH_3)_3$

- la tris(oxa-3 heptyl) amine de formule :

$N-(CH_2-CH_2-O-C_4H_9)_3$

- la tris (dioxa-3,6 heptyl) amine de formule :

$N-(CH_2-CH_2-O-CH_2-CH_2-O-CH_3)_3$

- la tris(trioxa-3,6,9 décyl) amine de formule :

$N-(CH_2-CH_2-O-CH_2-CH_2-O-CH_2-CH_2-O-CH_3)_3$

- la tris(dioxa-3,6 octyl) amine de formule :

$N-(CH_2-CH_2-O-CH_2-CH_2-O-C_2H_5)_3$

- la tris(trioxa-3,6,9 undécyl) amine de formule :

  $N-(CH_2-CH_2-O-CH_2-CH_2-O-CH_2-CH_2-O-C_2H_5)_3$

- la tris(dioxa- 3,6 nonyl) amine de formule :

  $N-(CH_2-CH_2-O-CH_2-CH_2-O-C_3H_7)_3$

- la tris(trioxa-3,6,9 dodécyl) amine de formule :

  $N-(CH_2-CH_2-O-CH_2CH_2-O-CH_2-CH_2-O-C_3H_7)_3$

- la tris(dioxa-3,6 décyl) amine de formule :

  $N-(CH_2-CH_2-O-CH_2-CH_2-O-C_4H_9)_3$

- la tris(trioxa-3,6,9 tridécyl amine de formule :

  $N-(CH_2-CH_2-O-CH_2-CH_2-O-CH_2-CH_2-O-C_4H_9)_3$

- la tris(tétraoxa-3,6,9, 12 tridécyl) amine de formule :

  $N-(CH_2-CH_2-O-(CH_2-CH_2-O)_3-CH_3)_3$

- la tris(dioxa-3,6 diméthyl-4 heptyl) amine de formule :

  $N-(CH_2-CH_2-O-CHCH_3-CH_2-O-CH_3)_3$

- la tris(dioxa-3,6 diméthyl-2,4 heptyl) amine de formule :

  $N-(CH_2-CHCH_3-O-CHCH_3-CH_2-O-CH_3)_3$.

On préfère utiliser parmi les composés de formule (I) : la tris(dioxaheptyl) amine (ou $TDA_1$).
La préparation de ces agents séquestrants est décrite dans la demande de Brevet Français 79 05438 publiée sous le N° 2450120.

Les éthers cycliques qui peuvent être mis en oeuvre dans le procédé selon l'invention comprennent des composés comme le dioxanne ou des éthers macrocycliques connus sous l'appellation générale d'"éthers couronnes" qui sont décrits dans le Brevet Français 69. 43879 publié sous le numéro 2.026.481.

On peut citer comme exemples d'éthers couronnes pouvant être utilisés selon l'invention :

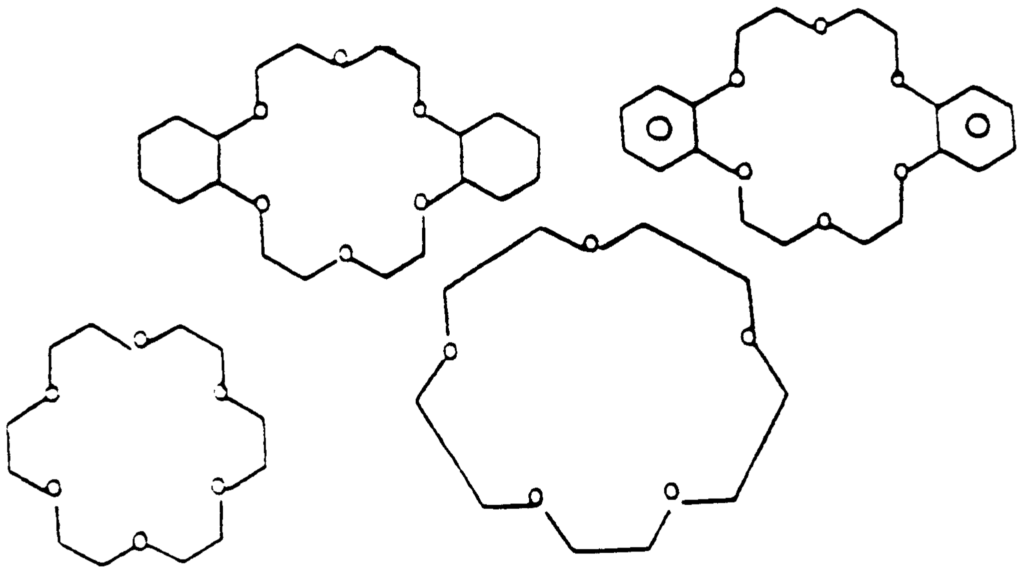

Une partie des composés de la troisième classe d'agents complexants sont décrits dans le Brevet Français 70.21079 publié sous le numéro 2.052.947. On peut citer comme exemples de composés de cette classe convenant pour la mise en oeuvre du procédé selon l'invention :

$CH_3- O - CH_2- CH_2- O - CH_2 - CH_2- OCH_3$

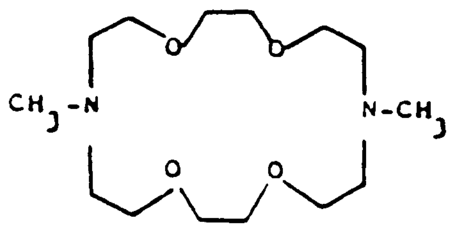

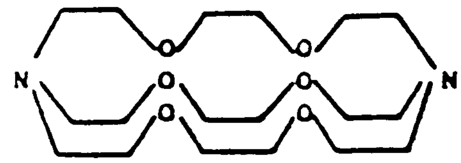

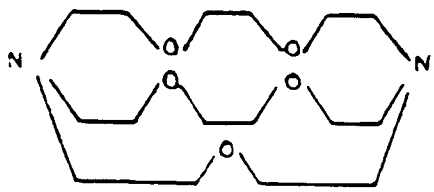

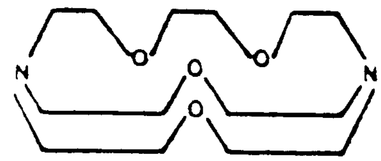

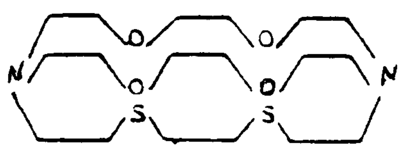

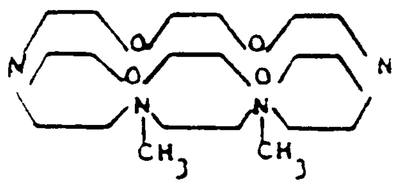

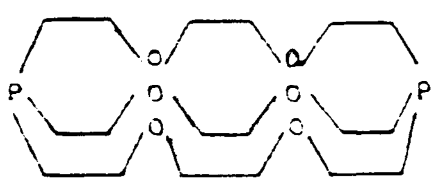

Selon un premier procédé préférentiel de mise en oeuvre de l'invention, on met en contact le tertiobutylate de sodium avec du dioxyde de carbone, on ajoute ensuite le phosgène ou le diphosgène ou le triphosgène.

L'agent complexant peut être introduit à tout moment, avant la carbonatation ou avant l'introduction du phosgène ou du diphosgène ou du triphosgène.

Selon un deuxième procédé préférentiel, le phosgène ou le diphosgène ou le triphosgène est tout d'abord introduit, puis on ajoute le pyrocarbonate de tertiobutyle, le complexant pouvant être ajouté à tout moment.

La réaction peut être réalisée dans tout solvant quelle que soit la nucléophilie du milieu. On peut citer parmi les solvants :

- les dérivés hydrocarbonés aromatiques tels que :
  . le benzène,
  . les xylènes,

. le toluène,
- les solvants oxygénés tels que le dioxanne et les éthers diméthyliques de l'éthylène glycol ou des polyéthylènes glycols et leur mélange, lesquels solvants peuvent jouer le rôle de complexant (s).

Selon un mode préféré de réalisation de l'invention on utilise une quantité molaire de dihalogénure de diacide ou équivalents comprise entre 0,3 et 1 fois, la quantité de tertiobutylate utilisée.

La température de réaction est avantageusement comprise entre -10 ° C et 60 ° C.

La présente invention sera plus complètement illustrée à l'aide des exemples suivants qui ne doivent pas être considérés comme limitatifs de l'invention.

Exemples 1 et 2 et essai comparatif A

**Mode opératoire type . Influence de la quantité de $TDA_1$**

Dans un réacteur de 250 ml, on charge
12,8 g de tertiobutyle de sodium (0,075 mole)
150 ml de toluène sec.

A température ambiante, on introduit du $CO_2$, ce qui provoque une exothermie. L'introduction est effectuée en trente minutes.
L'introduction du $CO_2$ est alors arrêtée et on refroidit le mélange réactionnel à 15 ° C, température à laquelle on ajoute dans un premier temps la $TDA_1$, puis du phosgène en 2 H 30. Le mélange réactionnel est très épais. On laisse ensuite remonter à 35 ° C et on agite 2 heures 30, puis éventuellement encore 1 heure à 55 ° C.

Après refroidissement, on ajoute 100 ml d'eau glacée. La phase organique lavée avec 3 x 100 ml d'eau, séchée sur 15 g de sulfate de sodium est évaporée à température ambiante. Le produit obtenu de couleur orangée est analysé par CPG.

| ESSAI | Durée d'introduction du phosgène | Durée à 35 ° C | Durée à 55 ° C | Quantité phosgène (moles) | TDA1 (ml) | RR % |
|---|---|---|---|---|---|---|
| Comparatif A | 2 h 30 | 2 h 30 | 1 h | 0, 102 | 0 | 31 |
| Exemple 1 | 2 h 30 | 2 h 30 | 1 h | 0,078 | 3 | 58 |
| Exemple 2 | 2 h 30 | 2 h 30 | 0 | 0,097 | 3 | 41 |

On entend par RR, le rendement sur le produit introduit, c'est-à-dire :

$$\frac{\text{quantité de produit obtenu (moles)}}{\text{quantité de tertiobutylate introduit (moles)}} \%$$

Exemple 3

**Influence de la température**

On opère comme dans l'exemple 1 en chargeant
0,075 mole de tertiobutylate de sodium
0,112 mole de phosgène
150 ml de toluène
3 ml de TDA 1 (0,0015 mole) La carbonatation est effectuée à 10 ° C, puis la température de réaction est amenée à 55 ° C et à cette température, on introduit le phosgène pendant 2 H 30 ; ensuite la réaction est poursuivie à cette température pendant 3 Heures.

On obtient un RR en dicarbonate de ditertiobutyle de 66 %

Exemple 4

**Utilisation du triphosgène**

On opère comme dans l'exemple 1 en chargeant :
0,13 mole de tertiobutylate de sodium (TBuONa)
0,0167 mole de triphosgène (soit 0,0501 mole de phosgène)
75 ml de toluène
0,015 mole de TDA 1
La carbonatation est effectuée à 10° C et après introduction du triphosgène, la réaction est poursuivie à 20° C pendant 5 h 30.

On obtient un RR en $BOC_2O$ de 51 % (dans cet exemple le RR est calculé par rapport au triphosgène engagé exprimé en phosgène).

Exemple 5

**Utilisation de diphosgène**

On opère comme dans l'exemple 1 en chargeant :
0,13 mole de tertiobutylate de sodium (TBuONa)
0,025 mole de diphosgène (soit 0,0500 mole de phosgène)
75 ml de toluène
0,015 mole de $TDA_1$
La carbonatation est poursuivie à 20° C pendant 5 h 30.

On obtient un RR en $BOC_2O$ de 50 % (dans cet exemple, le RR est calculé par rapport au diphosgène engagé exprimé en phosgène).

Exemple 6

Dans un réacteur de 250 ml, on charge 12,8 g de tBuONa (0,13 mole), et le toluène et à 5° C, on fait barboter pendant 30 minutes du $CO_2$, de manière à former le carbonate de tertiobutyle; après avoir stoppé l'introduction du $CO_2$, on ajoute 5 g de TDA1. 6,6 g de chlorure d'oxalyle (0,051 mole) sont coulées à 5°C en 15 minutes. On laisse remonter la température puis on laisse agiter pendant 4 h 30. Après traitement, on recueille 7,6 g d'une solution dans laquelle on dose, par CPG, 79 % de $BOC_2O$, ce qui représente un rendement de 54 % par rapport au chlorure d'oxalyle engagé.

Exemple 7

**Synthèse du $BOC_2O$ à partir de chlorure d'oxalyle en présence de diisopropylethyl amine**

Dans un réacteur de 250 ml, on charge 75 ml de toluène, 12,8 g de t.BuONa (130 mM), 3 ml de diisopropyléthylamine. A cette suspension, on ajoute à température ambiante du $CO_2$, pendant environ 30 mn, jusqu'à ce que le débit de $CO_2$ à la sortie soit identique au débit de l'entrée.

Après refroidissement à 5°C, on additionne en 20 mn, 6,4g de chlorure d'oxalyle (42,8 mM) en solution dans 30 ml de toluène, puis on laisse revenir le mélange à température ambiante, où il est agité pendant 5h.

Après traitement, on recueille 5,05 g d'un liquide dans lequel on dose par RMN 30 % de $BOC_2O$, ce qui représente un rendement de 10 % par rapport au chlorure d'oxalyle engagé.

Exemple B comparatif

**Phosgénation dans le toluène en présence de diaza-1,4 bicyclo (2,2,2) octane**

Dans un réacteur de 250 ml, on charge 7,5 g de tBuONa (75 mM), 1,7 g de diaza-1,4 bicyclo (2,2,2) octane (15 mM) et 75 ml de toluène. A cette suspension, on introduit à température ambiante du $CO_2$, pendant environ 30 mn, jusqu'à ce que le débit de $CO_2$ à la sortie soit identique au débit à l'entrée.
A température ambiante, on introduit pendant 2 h, 11,9 g de phosgène (120 mM), puis on purge à l'azote en chauffant le mélange réactionnel à 55° pendant 2 h.

Après refroidissement et traitement, on recueille 9,5 g d'un liquide blanc dans lequel on dose 4,6 % de $BOC_2O$, ce qui correspond à un rendement de 1% par rapport au t.butylate de sodium.

Exemple C comparatif

Dans un réacteur de 250 ml, on charge 12,8 g de tBuONa (0,13 mole), et le toluène et à 5°C, on fait barbotter pendant 30 minutes du $CO_2$, de manière à former le carbonate de tertiobutyle. 6,6 g de chlorure d'oxalyle (0,051 mole) sont coulées à 5° C en 15 minutes. On laisse remonter la température puis on laisse agiter pendant 4 h 30. Après traitement, on recueille 13,9g d'une solution dans laquelle on dose, par CPG, 2,6 % de $BOC_2O$, ce qui représente un rendement de 3,2 % par rapport au chlorure d'oxalyle engagé.

**Revendications**

1. Procédé de préparation de dicarbonate (ou pyrocarbonate) de dialcoyle secondaire ou tertiaire où l'on met en contact un carbonate mixte d'alcoyle et de métal alcalin avec un dihalogénure de diacide choisi parmi les halogénures de carbonyles et leurs di- ou trimères et les halogénures d'oxalyle caractérisé en ce que
   - ledit contact est mené en présence d'un agent complexant choisi parmi ceux possédant au moins une fonction amine ou possédant une fonction éther comportant au moins une autre fonction éther et parmi les cryptants,
   - avec la condition que lorsque ledit dihalogénure de diacide est le phosgène, le diphosgène, et/ou le triphosgène, l'agent complexant n'est ni le dioxanne, ni une mono amine tertiaire, ni une amine choisie parmi le 1,4-diazabicyclo (2,2,2) octane, le 1,8-diazabicyclo (5,4,0) undecene-7 l'hexaméthyltétramine, la N-méthylpipéridine, la N-éthylpipéridine, la N-méthylmorpholine, la morpholine, la N,N'-dialcoylpiperazine, la pyridine, la quinoléine et l'isoquinoléine.

2. Procédé selon la revendication 1, caractérisé en ce que ledit dihalogénure de diacide est le chlorure d'oxalyle.

3. Procédé selon l'une des revendications 1 et 2, caractérisé en ce que ledit agent complexant est choisi parmi les polyéthers oxygénés ou soufrés, linéaires, cycliques ou macrocycliques.

4. Procédé selon l'une des revendications 1 à 3, caractérisé en ce que les amines tertiaires oxygénées répondent à la formule :

   $$N\text{-}[\text{-}CHR_1 - CHR_2 - O - (CHR_3 - CHR_4 - O\text{-})_n\text{-} R_5\ ]_3$$

   dans laquelle n est un nombre entier supérieur ou égal à 0 et inférieur ou égal à 10 ($0 \leqq n \leqq 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $-C_mH_{2m}\text{-}\Phi$, ou $C_mH_{2m+1}\text{-}\Phi\text{-}$, m étant compris entre 1 et 12.

5. Procédé selon l'une des revendications 1 à 3 caractérisé en ce que les agents complexants répondent aux formules II suivantes :

   $$R_{10}\text{-}Y[A - D]_p A - Y - R_{10} \qquad IIa$$

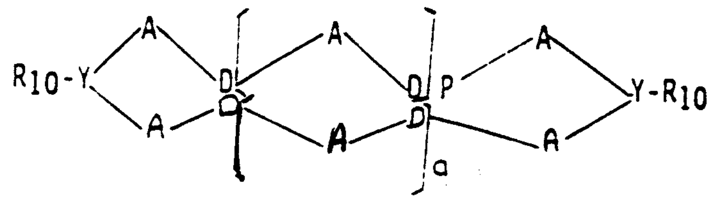 IIb

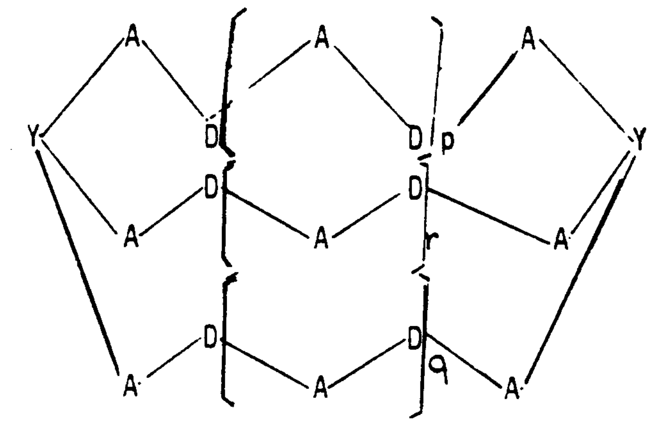

dans lesquelles

- Y représente O (divalent) , N ou P (trivalent) dans les formules IIa et IIb et N ou P dans la dernière formule
- A représente un groupement alkylène ayant de 1 à 3 atomes de carbone.
- D représente O, S ou N-$R_{11}$, $R_{11}$ représente un radical alkyle ayant de 1 à 6 atomes de carbone.
- $R_{10}$ représente un radical alkyle ayant 1 à 6 atomes de carbone.
- p, q et r identiques ou différents sont des nombres entiers compris entre 1 et 5.
- lorsque dans les formules ci-dessus la valence des atomes Y n'est pas satisfaite, des radicaux alkyles $R_{10}$ satisfont alors les dites valences

**6.** Procédé selon la revendication 4 caractérise en ce que l'amine oxygénée est la tris(dioxa-3,6heptyl)-amine

**7.** Procédé selon les revendications 1 à 3 caractérisé en ce que le polyéther est le diéther méthylique du diéthylène glycol.

**8.** Procédé selon l'une des revendications 1 à 7, caractérisé en ce que la réaction est réalisée dans un solvant aromatique, de préférence dans le toluène.

**9.** Procédé selon l'une des revendications 1 à 8, caractérisé en ce que ledit carbonate mixte d'alcoyle et de métal alcalin est un sel de lithium ou de sodium

**10.** Procédé selon la revendication 2, caractérisé en ce que le polyéther est le dioxanne.

**Claims**

**1.** Process for the preparation of secondary or tertiary dialkyl dicarbonate (or pyrocarbonate), wherein a mixed alkali metal alkyl carbonate is placed in contact with a diacid dihalide chosen from carbonyl halides and their di- or trimers and oxalyl halides, characterized in that

- the said contact is conducted in the presence of a complexing agent chosen from those which have at least one amine functional group or have an ether functional group comprising at least one other ether functional group and from cryptants,
- with the condition that when the said diacid dihalide is phosgene, diphosgene and/or triphosgene, the complexing agent is neither dioxane nor a tertiary monoamine nor an amine chosen from 1,4-diazabicyclo[2.2.2]octane, 1,8-diazabicyclo[5.4.0]-7-undecene, hexamethyltetramine, N-methylpiperidine, N-ethylpiperidine, N-methylmorpholine, morpholine, N,N'-dialkylpiperazine, pyridine, quinoline and isoquinoline.

**2.** Process according to Claim 1, characterized in that the said diacid dihalide is oxalyl chloride.

**3.** Process according to either of Claims 1 and 2, characterized in that the said complexing agent is chosen from linear, cyclic or macrocyclic oxygen-containing or sulphur-containing polyethers.

**4.** Process according to one of Claims 1 to 3, characterized in that the oxygen-containing tertiary amines correspond to the formula:

$$N\text{-}[\text{-}CHR_1\text{-}CHR_2\text{-}O\text{-}(CHR_3\text{-}CHR_4\text{-}O\text{-})_n\text{-}R_5]_3$$

in which n is an integer greater than or equal to 0 and smaller than or equal to 10 ($0 \leqq n \leqq 10$), $R_1$, $R_2$, $R_3$ and $R_4$, which are identical or different, denote a hydrogen atom or an alkyl radical containing from 1 to 4 carbon atoms and $R_5$ denotes an alkyl or cycloalkyl radical containing from 1 to 12 carbon atoms, a phenyl radical or a radical of formula $-C_mH_{2m}-\Phi$, or $C_mH_{2m+1}-\Phi-$, m being between 1 and 12.

**5.** Process according to one of Claims 1 to 3, characterized in that the complexing agents correspond to the following formulae II:

$$R_{10}\text{-}Y[A - D]_p A - Y - R_{10} \qquad IIa$$

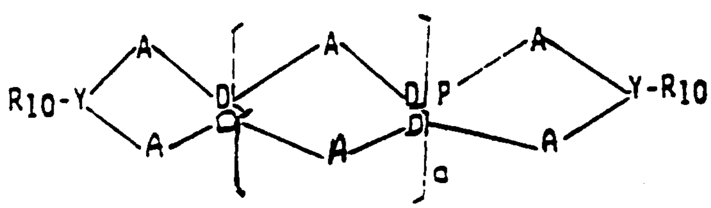

IIb

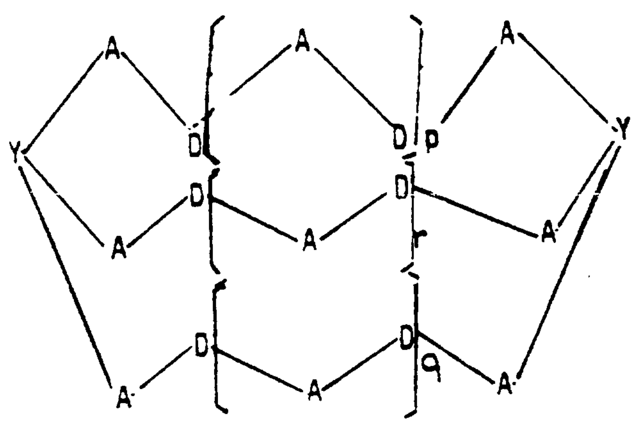

in which

- Y denotes O (divalent), N or P (trivalent) in formulae IIa and IIb and N or P in the last formula,
- A denotes an alkylene group containing from 1 to 3 carbon atoms,
- D denotes O, S or N-$R_{11}$, $R_{11}$ denotes an alkyl radical containing from 1 to 6 carbon atoms,
- $R_{10}$ denotes an alkyl radical containing 1 to 6 carbon atoms,
- p, q and r, which are identical or different, are integers included between 1 and 5,
- when in the above formulae the valency of the atoms Y is not satisfied, alkyl radicals $R_{10}$ then satisfy the said valencies.

**6.** Process according to Claim 4, characterized in that the oxygen-containing amine is tris(3,6-dioxaheptyl)amine.

7. Process according to Claims 1 to 3, characterized in that the polyether is diethylene glycol methyl diether.

8. Process according to one of Claims 1 to 7, characterized in that the reaction is carried out in an aromatic solvent, preferably in toluene.

9. Process according to one of Claims 1 to 8, characterized in that the said mixed alkali metal alkyl carbonate is a lithium or sodium salt.

10. Process according to Claim 2, characterized in that the polyether is dioxane.

**Patentansprüche**

1. Verfahren zur Herstellung von sekundärem oder tertiärem Dialkyldicarbonat (oder -pyrocarbonat), wobei ein gemischtes Alkyl- und Alkalimetallcarbonat mit einem Dihalogenid einer zweiwertigen Säure, ausgewählt aus den Carbonylhalogeniden und ihren Di- oder Trimeren und den Oxalylhalogeniden in Kontakt gebracht werden, dadurch gekennzeichnet, daß
   - die Kontaktierung in Gegenwart eines Komplexierungsmittels, das aus solchen, die wenigstens eine Aminfunktionalität oder eine Etherfunktionalität besitzen und wenigstens eine weitere Etherfunktionalität aufweisen, und aus den Kryptanden ausgewählt ist, durchgeführt wird,
   - mit der Maßgabe, daß das Komplexierungsmittel weder Dioxan, noch ein tertiäres Monoamin, noch ein Amin ist, ausgewählt aus 1,4-Diazabicyclo-(2,2,2)-oktan, 1,8-Diazabicyclo-(5,4.0)-7-undecen, Hexamethyltetramin, N-Methylpiperidin, N-Ethylpiperidin, N-Methylmorpholin, Morpholin, N, N'-Dialkylpiperazin, Pyridin, Chinolin und Isochinolin, wenn das Dihalogenid der zweiwertigen Säure Phosgen, Diphosgen und/oder Triphosgen ist.

2. Verfahren nach Anspruch 1, dadurch gekennzeichnet, daß das Dihalogenid der zweiwertigen Säure Oxalylchlorid ist.

3. Verfahren nach Anspruch 1 oder 2, dadurch gekennzeichnet, daß das Komplexierungsmittel ausgewählt ist aus dem sauerstoffhaltigen oder schwefelhaltigen, geradkettigen, cyclischen oder makrocyclischen Polyethern.

4. Verfahren nach einem der Ansprüche 1 bis 3, dadurch gekennzeichnet, daß die sauerstoffhaltigen tertiären Amine der Formel:

   $$N-[-CHR_1-CHR_2-O-(CHR_3-CHR_4-O-)_n-R_5]_3$$

   entsprechen, in der n eine ganze Zahl größer oder gleich 0 und kleiner oder gleich 10 ist ($0 \leqq n \leqq 10$), $R_1$, $R_2$, $R_3$ und $R_4$ gleich oder verschieden sind und ein Wasserstoffatom oder einen Alkylrest mit 1 bis 4 Kohlenstoffatomen darstellen und $R_5$ einen Alkyl- oder Cycloalkylrest mit 1 bis 12 Kohlenstoffatomen, einen Phenylrest oder einen Rest der Formel
   $C_mH_{2m}$-Φ, oder $C_mH_{2m+1}$-Φ-, darstellt, wobei m im Bereich von 1 bis 12 liegt.

5. Verfahren nach einem der Ansprüche 1 bis 3, dadurch gekennzeichnet, daß die Komplexierungsmittel den folgenden Formeln II entsprechen:

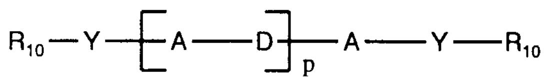

IIa

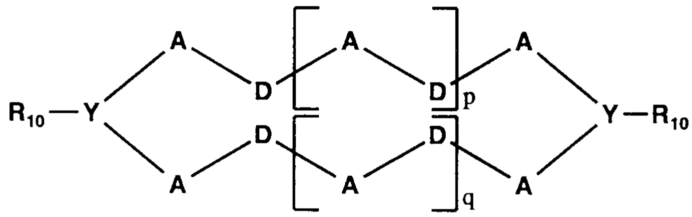

IIb

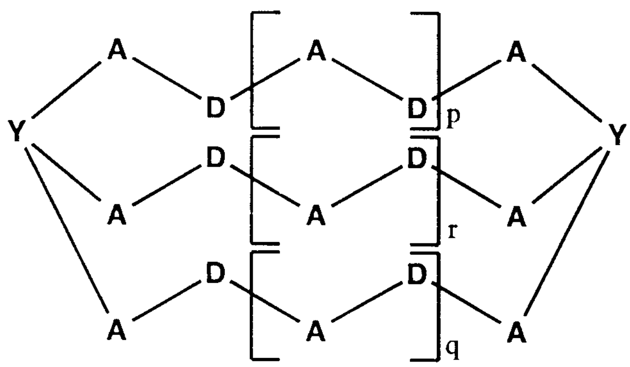

in denen

- Y (zweiwertigen) O, (dreiwertigen) N oder P in den Formeln IIa und IIb und N oder P in der letzten Formel darstellen,
- A eine Alkylengruppe mit 1 bis 3 Kohlenstoffatomen darstellt,
- D O, S oder N-$R_{11}$ darstellt, wobei $R_{11}$ ein Alkylrest mit 1 bis 6 Kohlenstoffatomen ist,
- $R_{10}$ einen Alkylrest mit 1 bis 6 Kohlenstoffatomen darstellt,
- p, q und r gleich oder unterschiedlich und ganze Zahlen im Bereich von 1 bis 5 sind;
- wenn in den vorstehenden Formeln die Valenz der Atome Y nicht gesättigt ist, so sättigen die Alkylreste $R_{10}$ diese Valenzen ab.

**6.** Verfahren nach Anspruch 4, dadurch gekennzeichnet, daß das sauerstoffhaltige Amin Tris(3,6-dioxaheptyl)-amin ist.

**7.** Verfahren nach einem der Ansprüche 1 bis 3, dadurch gekennzeichnet, daß der Polyether Diethylenglykoldimethylether ist.

**8.** Verfahren nach einem der Ansprüche 1 bis 7, dadurch gekennzeichnet, daß die Reaktion in einem aromatischen Lösungsmittel, vorzugsweise in Toluol, durchgeführt wird.

**9.** Verfahren nach einem der Ansprüche 1 bis 8, dadurch gekennzeichnet, daß das gemischte Alkyl- und Alkalimetallcarbonat ein Lithium- oder Natriumsalz ist.

**10.** Verfahren nach Anspruch 2, dadurch gekennzeichnet, daß der Polyether Dioxan ist.